Europäisches Patentamt

European Patent Office

Office européen des brevets

(19)

(11) Veröffentlichungsnummer: **0 377 831**

**A1**

(12) EUROPÄISCHE PATENTANMELDUNG

(21) Anmeldenummer: 89122750.6

(22) Anmeldetag: 09.12.89

(51) Int. Cl.5: **C07H 15/04**

(30) Priorität: 17.12.88 DE 3842541

(43) Veröffentlichungstag der Anmeldung:
18.07.90 Patentblatt 90/29

(84) Benannte Vertragsstaaten:
GR

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Hill, Karlheinz, Dr.**
**Am Hasenbusch 1**
**D-4006 Erkrath(DE)**
Erfinder: **Biermann, Manfred, Dr.**
**Markscheiderhof 25**
**D-4330 Mülheim(DE)**

(54) Verfahren zur Herstellung von oberflächenaktiven Alkylglucosiden.

(57) Bei dem Verfahren nach der Umacetalisierungsmethode mit Butanol wird zunächst Glucose mit Butanol unter Säurekatalyse umgesetzt, das Reaktionswasser abdestilliert, und dann die saure Lösung des Butylglucosids in Butanol zu einer auf 100 - 125 °C vorgewärmten Mischung eines längerkettigen ($C_{12}$-$C_{18}$) primären Alkohols mit weiterem sauren Katalysator unter Abdestillation des Butanols hinzugegeben. Nach der an sich üblichen Aufarbeitung wird ein im Alkalischen farbstabiles Produkt mit hohen Anteilen an $C_{12}$-$C_{18}$-Alkylmonoglucosid und geringen Anteilen an Butylglucosid erhalten.

EP 0 377 831 A1

EP 0 377 831 A1

## Verfahren zur Herstellung von oberflächenaktiven Alkylglucosiden

Die Erfindung betrifft ein neues, verbessertes Verfahren zur Herstellung von Alkylglucosiden nach der Umacetalisierungsmethode mit Butanol.

Die Herstellung oberflächenaktive Alkylglucosidverbindungen enthaltender Reaktionsprodukte ist bereits in zahlreichen Druckschriften beschrieben. Alkylglucoside sind bekanntlich Acetale aus Zuckern und monofunktionellen Alkoholen, die zur Herstellung von Wasch- und Reinigungsmitteln verwendet werden.

Die oberflächenaktiven Alkylglucoside mit im wesentlichen $C_{12}$-$C_{18}$-Alkyl- bzw Alkenylresten gehören zum Typ der nichtionischen Tenside. Dabei besitzen überwiegend Alkylmonoglucoside enthaltende Reaktionsprodukte besondere Bedeutung. Je nach Herstellung sind in den Reaktionsgemischen auch untergeordnete Mengen an Acetalen enthalten, die den Rest des längerkettigen monofunktionellen Alkohols an Oligosaccharidreste gebunden enthalten. Der Begriff Alkylglucosid umfaßt damit sowohl die Alkylmonoglucoside als auch Alkyloligo- bzw. Alkylpolyglucoside, sofern es nicht ausdrücklich auf die jeweiligen strukturellen Unterschiede ankommt.

Der auf die längerkettigen monofunktionellen Alkohole zurückgehende Bestandteil der Alkylglucoside - der Alkylrest - führt auf entsprechende Alkohole natürlichen und/oder synthetischen Ursprungs zurück. Das für die Praxis wichtigste Saccharid-Ein satzmaterial sind in der Natur vielfältig zur Verfügung stehende wasserfreie Polyglucoseverbindungen, deren einzelne Glucoseeinheiten in $\alpha$-glucosidischer Verknüpfung vorliegen. Das wichtigste Einsatzmaterial natürlichen Ursprungs dieser Art ist die Stärke, die weltweit durch Nutzpflanzen unterschiedlichster Art - beispielsweise Kartoffeln, Mais, Tapioka, Reis und dergleichen -gebildet wird und zum Monosaccharid, d. h. zur Glucose abgebaut werden kann. Die Glucose kann dann als wasserfreies Material oder als Glucosehydrat der Acetalisierung zugeführt werden.

Aus den 60er und 70er Jahren stammt eine Vielzahl von Vorschlägen zu verbesserten Herstellungsverfahren für Alkylglucoside entweder durch direkte Umsetzung der Glucose mit einem Überschuß des Fettalkohols und einer Säure als Katalysator (Direktsynthese) oder unter Mitverwendung eines niederen Alkohols oder Glykols als Lösungsmittel und Reaktionspartner (Umacetalisierung).

So wird beispielsweise in der US-Patentschrift 3 547 828 die Herstellung eines ternären Gemisches aus Alkyloligoglucosiden, Alkylmonoglucosiden und den entsprechenden langkettigen, monofunktionellen $C_{11}$-$C_{32}$-Alkanolen nach dem Umacetalisierungsverfahren mit Butanol beschrieben. Dabei wird zunächst die Glucose mit Butanol und einem saurem Katalysator, z. B. Schwefelsäure, zu Butylglucosid umgesetzt, wobei das Reaktionswasser bei der Rückflußtemperatur abgeschieden wird. Man benutzt dabei 2 bis 6 Mol Butanol pro Mol Glucose. Danach wird der Fettalkohol in Mengen von 0,5 bis 4 Mol pro Mol Glucose hinzugegeben und das überschüssige Butanol sowie das bei der Umacetalisierungsreaktion gebildete Butanol abdestilliert. Die Umacetalisierungsreaktion wird gegebenenfalls abgebrochen, so daß noch Teile des Butylglucosids im Reaktionsgemisch verbleiben. Auf diese Weise lassen sich Produkte mit niedriger Viskosität herstellen. Anschließend wird der saure Katalysator durch Zugabe von Natriumhydroxidlösung neutralisiert. Danach wird im Vakuum der überschüssige Fettalkohol zum größten Teil bis auf das gewünschte Niveau entfernt, meist auf Werte von weniger als 2 Gew.-%.

Nach der Lehre der europäischen Patentanmeldung 92 875 wird das Umacetalisierungsverfahren mit Butanol zur Herstellung von langkettigen Alkylglucosiden so gesteuert, daß noch eine Restmenge an Butylglucosiden von weniger als 10 Gew.-% im Verfahrensprodukt enthalten ist. Auf diese Weise wird die Bildung der langkettigen Alkyloligoglucoside mit höherem Oligomerisierungsgrad, d. h. mit 6 und mehr Glucoseeinheiten im Molekül verringert. Die so erhaltenen Produkte bestehen im wesentlichen aus Alkylmonoglucosid und Alkyloligoglucosiden, wobei der Mengenanteil der Alkylmonoglucoside maximal 60 Gew.-% und der durchschnittliche Oligomerisierungsgrad 1,5 bis 3 beträgt. Der Anteil an kurzkettigen Alkylglucosiden, insbesondere Butylglucosiden, liegt unterhalb 10 %, der Anteil nicht umgesetztem Fettalkohol soll unter 2 % liegen. Zur destillativen Entfernung des Fettalkohols wird die Benutzung eines Dünnschichtverdampfers empfohlen.

Die nach diesen Verfahren hergestellten Produkte besitzen den Nachteil, daß sie nicht alkalifarbstabil sind.

Die Erfindung geht von der Aufgabe aus, alkalifarbstabile oberflächenaktive Alkylglucosidverbindungen mit hohen Raum-Zeit-Ausbeuten sowie hohen Anteilen an Alkylmonoglucosid und geringen Anteilen an Niedrig-Alkyl-Glucosid herzustellen.

Gegenstand der Erfindung ist dementsprechend ein Verfahren zur Herstellung von oberflächenaktiven Alkylglucosiden mit überwiegendem Gehalt an Alkylmonoglucosiden aus Glucose und längerkettigen monofunktionellen Alkoholen nach der Umacetalisierungsmethode mit Butanol, gekennzeichnet durch Eintragung einer sauren Lösung (A) von Butylglucosid in Butanol in (B) einen auf 100 bis 125 $^\circ$C vorgeheizten

2

längerkettigen monofunktionellen Alkohol, der allein oder im Gemisch mit einem sauren Katalysator vorliegt.

Das als intermediäres Niedrig-Alkyl-Glucosid eingesetzte Butylglucosid kann nach jedem der bekannten, zum Stand der Technik gehörenden Verfahren hergestellt werden, nach dem eine Lösung (A) von Butylglucosid in Butanol, die im Gemisch mit einem sauren Katalysator vorliegt, erhalten wird. Vorzugsweise wird dieses als saure Lösung (A) bezeichnete Gemisch nach dem in der deutschen Patentanmeldung 37 23 826 beschriebenen Verfahren erhalten. Die dort beschriebene Herstellungsmethode von Butylglucosid umfaßt die folgenden Verfahrensschritte:

a) Butanol wird zusammen mit einem sauren Katalysator im Reaktionsgefäß vorgelegt;

b) die Butanolmenge wird so gewählt, daß sie, bezogen auf 1 Mol der Glucose, 4 bis 10 Mol, vorzugsweise 6 bis 8 Mol, beträgt;

c) von der Butanolmenge wird ein Teil, vorzugsweise etwa die Hälfte des Butanols zusammen mit dem Katalysator vorgelegt und die andere Hälfte zur Suspendierung der Glucose verwen-det;

d) als Katalysator wird eine sauer reagierende Verbindung, insbesondere eine Säure aus der Gruppe bestehend aus Schwefelsäure, Phosphorsäure, Paratoluolsulfonsäure und sulfosauren Ionenaustauscherharzen, in einer Menge von vorzugsweise 0,002 bis 0,02 Mol pro Mol der eingesetzten Glucose verwendet;

e) Erhitzen des Butanol/Katalysator-Gemisches auf Rückflußtemperatur;

f) Zugabe einer vorzugsweise vorgewärmten Suspension des übrigen Butanols und der wasserhaltigen oder wasserfreien Glucose unter portionsweiser oder kontinuierlicher Zudosierung unter Rühren so, daß das Reaktionsgemisch praktisch klar bleibt;

g) unmittelbares Abdestillieren des freiwerdenden Wassers als Butanol/Wasser-Gemisch;

h) es wird vorzugsweise ein leichtes Vakuum von etwa 800 -950 mbar während oder nach der Zudosierung der Butanol/Glucose-Mischung angelegt und unter weiterer Wärmezufuhr und Rühren die Abdestillation des Reaktionswassers beendet.

Die im Gemisch mit dem sauren Katalysator anfallende Lösung (A) aus Butylglucosid in Butanol kann ohne weitere Aufarbeitung, insbesondere ohne Abreicherung an Butanol, der Umacetalisierung zugeführt werden.

Diese zweite Reaktionsstufe wird bevorzugt bei Temperaturen dicht unterhalb der ersten Reaktionsstufe durchgeführt, wobei hier Temperaturen im Bereich von 100 bis 120 °C besonders bevorzugt sein können

Als Alkoholkomponente für die zweite Reaktionsstufe sind längerkettige Alkohole bzw. Alkoholgemische natürlichen und/oder synthetischen Ursprungs, insbesondere primäre Alkanole, geeignet. Besondere Bedeutung kann den nachwachsenden Fettalkoholen zukommen, die durch Reduktion der Fettsäuren natürlichen Ursprungs erhalten werden können. Genauso sind aber entsprechende Alkohole bzw. Alkoholgemische synthetischen Ursprungs, wie zum Beispiel die Oxoalkohole, die nach der Methode der Oxosynthese bzw. Hydroformylierung erhalten werden, geeignet. Die Auswahl der jeweiligen Kettenlänge der eingesetzten Alkohole bzw. Alkoholgemische wird durch die jeweils geforderten tensidischen Eigenschaften der Alkylglucoside bestimmt. Für breite Anwendungsgebiete sind hier $C_{12}$-$C_{18}$-Kettenlängen besonders geeignet.

Vorzugsweise wird bei der Umacetalisierung derselbe Katalysator wie bei der Herstellung des intermediären sauren Butylglucosid/Butanol-Gemisches (A) wiederum in einer Menge von 0,002 bis 0,02 Mol pro Mol der eingesetzten Glucose verwendet. Besonders bevorzugt ist der Einsatz von Paratoluolsulfonsäure.

Die erfindungsgemäße Verfahrensführung sieht eine vorzugsweise kontinuierliche Zugabe des ersten Reaktionsproduktes, des Butylglucosids, zum vorgeheizten längerkettigen Alkohol bzw. Alkoholgemisch vor, so daß die längerkettigen Alkohole während des gesamten Verfahrensschrittes im beträchtlichen Überschuß bezüglich Butylglucosid vorhanden sind. Hierdurch wird die Bildung der insbesondere gewünschten hohen Ausbeuten an Monoalkylglucosid stark gefördert. Aus diesem Grunde können im erfindungsgemäßen Verfahren die für die Umacetalisierung einzusetzenden Alkoholmengen im Vergleich mit bereits bekannten Verfahren reduziert werden, ohne daß die Ausbildung der gewünschten hohen Anteile an Monoalkylglucosid negativ beeinflußt wird. Erfindungsgemäß ist es dementsprechend bevorzugt, im gesamten Ansatz des zweiten Reaktionsgemisches Molverhältnisse von eingesetzter Saccharidverbindung (bezogen auf wasserfreie Glucose) zum längerkettigen Alkohol im Bereich von 1 : 2,5 bis 7, insbesondere im Bereich von etwa 1 : 3 bis 6 zu verwenden. Hier liegt für den in der Regel nachfolgenden Verfahrensschritt der Abreicherung des zweiten Reaktionsproduktes an freiem längerkettigen Alkohol eine wichtige Verfahrensverbesserung vor.

Die Zugabe der sauren Butylglucosid/Butanol-Lösung (A) erfolgt im Vakuum, bei 50 bis 10 mbar oder bei stufenweiser Reduktion des Druckes von 900 auf 10 mbar, wobei gleichzeitig Butanol aus dem Reaktionsgemisch entfernt wird.

Das nach der Umacetalisierung anfallende Reaktionsprodukt wird in an sich bekannter Weise weiter gereinigt und aufgearbeitet, wie es im einzelnen in der eingangs geschilderten Literatur und insbesondere auch in der deutschen Patentanmeldung 37 23 826 geschildert ist.

In Betracht kommt hier dementsprechend zunächst die Neutralisation des sauer katalysierten Reaktionsproduktes. Geeignet sind hierfür organische oder anorganische basische Alkali-, Erdalkali-oder Aluminium- bzw. Alkali/Aluminiumverbindungen. Zweckmäßigerweise werden dabei pH-Werte von wenigstens 8, vorzugsweise von etwa 9 - 10 eingestellt. Zur Neutralisation des Katalysators sind insbesondere Magnesiumverbindungen, beispielsweise Organomagnesiumverbindungen wie Magnesiumalkoholate oder anorganische Magnesiumverbindungen wie Magnesiumoxid bzw. Magnesiumhydroxid geeignet. Ein brauchbares Neutralisationsmittel ist aber auch Zeolith NaA, insbesondere in Abmischung mit Calciumhydroxid. Die Einstellung alkalischer Werte in dieser Neutralisationsstufe stellt die Farbstabilität des Tensids im Alkalischen bei seiner späteren Verarbeitung sicher.

Die Abdestillation des Fettalkoholüberschusses kann in bekannten produktschonenden Vakuumdestillationsvorrichtungen erfolgen. Geeignet ist hier insbesondere der Einsatz von Dünnschicht- und/oder Fallfilmverdampfern.

Das Reaktionsprodukt bildet im erkalteten Zustand eine wachsartige Masse, die in an sich bekannter Weise zur besseren Handhabbarkeit in wäßrige Pasten mit Wirkstoffgehalten von 45 bis 60 % überführt wird. In dieser Form kann insbesondere - soweit erforderlich - durch eine einfache Bleiche mit Wasserstoffperoxid oder einer organischen Persäure bzw. entsprechenden Persäuresalzen eine zusätzliche Farbkorrektur und Farbaufhellung er folgen. Für die meisten Anwendungszwecke des erfindungsgemäß hergestellten Alkylglucosids zur Herstellung von Wasch- und Reinigungsmitteln ist die direkt mit dem Verfahren erzielte Produktqualität jedoch völlig ausreichend.

Die im erfindungsgemäßen Verfahren anfallenden Stoffgemische entsprechen in ihrer Zusammensetzung weitgehend der Offenbarung der deutschen Patentanmeldung 37 23 826, auf deren Angaben hier einfachheitshalber verwiesen wird.

Außerdem umfaßt die Erfindung Erzeugnisse, die im wesentlichen aus 5 Komponenten bestehen und sich von den in der deutschen Patentanmeldung 37 23 826 beschriebenen Fünfer-Kombinationen dahingehend unterscheiden, daß der Bereich des Gehalts an Butylglucosid verringert und der Gehalt an Alkylmonoglucosid entsprechend erhöht ist. Vorzugsweise enthalten diese Produkte 0,5 bis 5, insbesondere 2,5 bis 4 Gew.-% Fettalkohol, 55 bis 90, vorzugsweise 60 bis 90 -Gew-% Alkylmonoglucosid, 2 bis 22, vorzugsweise 3 bis 20 Gew.-% Alkyloligoglucosid, 4 bis 25 Gew.-% Polyglucose und 3 bes 10, vorzugsweise 5 bis 7 Gew-% Butylglucosid. Dabei setzt sich die Gesamtmenge an Alkylmonoglucosid und Alkyloligoglucosid derart zusammen, daß der mittlere Oligomerisierungsgrad x 1,1 bis 1,4, vorzugsweise etwa 1,3 beträgt.

## BEISPIELE

Beispiel 1
$\underline{\hspace{1cm}}$

Dieses Beispiel beschreibt das erfindungsgemäße Verfahren im Labormaßstab

Es wurden 222 g (3 Mol) n-Butanol in einem 2-Liter-Mehrhalskolben mit Rührer, Thermometer, Tropftrichter und Destillationsaufsatz zur Wasserabscheidung vorgelegt und dazu 2,2 g (11,2 mMol) Paratoluolsulfonsäure als Katalysator gegeben. Die Mischung wurde auf 110 °C erhitzt. Anschließend wurde eine Suspension von 180 g (1 Mol) wasserfreier Glucose in weiteren 222 g (3 Mol) n-Butanol portionsweise, und zwar in 10 Portionen in Abständen von jeweils 5 Minuten, hinzu dosiert. Dabei bildete das Reaktionsgemisch eine klare Phase aus. Während des Dosierungsvorganges wurde die Hauptmenge des entstehenden Reaktionswassers zusammen mit Butanol bei Normaldruck abdestilliert (Destillatmenge: 90,7 g, Wassergehalt 14,3 % bestimmt nach Karl Fischer).

Dieses Reaktionsgemisch wurde innerhalb 75 Minuten kontinuierlich zu einer auf ca. 105 °C vorgewärmten Mischung aus 1164 g (6 Mol) eines $C_{12}/C_{14}$-Fettalkohols (Gemisch aus ca. 75 Gew.-% Dodecanol und ca. 25 Gew.-% Tetradecanol) und 1 g (5,1 mMol) Paratoluolsulfonsäure gegeben, wobei gleichzeitig Butanol weiter abdestilliert wurde. Dazu wurde zunächst der Druck auf 800 mbar eingestellt und anschließend allmählich auf 10 mbar reduziert (Destillatmenge 334 g).

Nach der Abtrennung des Butanols wurde das Gemisch noch 30 Minuten lang bei 110 °C und Normaldruck gerührt und dann auf 90 °C abgekühlt. Die Desaktivierung des Katalysators erfolgte durch Zusatz von 2,5 (22,0 mMol) Magnesiumethylat, wozu man weitere 30 Minuten bei 90 °C rührte (pH-Wert: 9 - 10). Nach Filtration bei 90 °C durch eine beheizte Nutsche wurde das Produkt zur Abtrennung des überschüssigen Fettalkohols im Vakuum bei 0,01 mbar und einer Sumpftemperatur von maximal 160 °C destilliert. Die Destillatmenge betrug 1040 g, der Destillationsrückstand, also das Endprodukt, betrug 302 g. Dieser Rückstand wurde bei 70 bis 80 °C mit Wasser zu einer 60 %igen Paste verarbeitet.

Lovibond-Farbwerte des Produktes (40 %ig in Wasser/Isopropanol): 5,9 (gelb) und 1,4 (rot)

| Zusammensetzung des Produktes (Bestimmung mit GC und HPLC): | |
| --- | --- |
| restlicher Fettalkohol | 3,5 Gew.-% |
| Butylglucosid | 3 Gew.-% |
| Dodecyl/Tetradecylmonoglucosid | 64,5 Gew.-% |
| Dodecyl/Tetradecyloligoglucosid (hauptsächlich Maltosid) | 17 Gew.-% |
| Polyglucose (MG ca. 2500) | ca. 9 Gew.-% |

Beispiel 2

Verfahren unter Verwendung von wasserhaltiger Glucose

Ansatz und Verfahrensdurchführung wie in Beispiel 1; als Saccharideinsatzmaterial wurden jedoch 198 g Dextrose (Glucose mit 1 Mol Wasser) eingesetzt. Abgesehen davon, daß bei der Acetalisierung mit Butanol die zusätzliche Wassermenge von ca. 18 g abdestilliert wurde, waren keine wesentlichen Unterschiede gegenüber Beispiel 1 festzustellen.

## Ansprüche

1. Verfahren zur Herstellung von oberflächenaktiven Alkylglucosiden mit überwiegendem Gehalt an Alkylmonoglucosiden aus Glucose und längerkettigen monofunktionellen Alkoholen nach der Umacetalisierungsmethode mit Butanol, gekennzeichnet durch Eintragung einer sauren Lösung (A) von Butylglucosid in Butanol in (B) einen auf 100 bis 125 °C vorgeheizten längerkettigen monofunktionellen Alkohol, der allein oder im Gemisch mit einem sauren Katalysator vorliegt.

2. Verfahren nach Anspruch 1, dadurch gekennzeichnet, daß ein primärer monofunktioneller Alkohol mit $C_{12}$- bis $C_{18}$-Alkyl-bzw. Alkenylketten in Mengen von 2,5 bis 7 Mol pro Mol Glucose verwendet wird.

3. Verfahren nach Anspruch 1 oder 2, dadurch gekennzeichnet, daß als Katalysator eine Säure aus der Gruppe bestehend aus Schwefelsäure, Phosphorsäure, Paratoluolsulfonsäure und sulfosauren Ionenaustauscherharzen in einer Menge von 0,002 bis 0,02 Mol pro Mol der eingesetzten Glucose verwendet wird.

4. Verfahren nach einem oder mehreren der Ansprüche 1 bis 3, dadurch gekennzeichnet, daß die Lösung (A) von Butylglucosid in Butanol einen sauren Katalysator in einer Menge von 0,002 bis 0,02 Mol pro Mol der eingesetzten Glucose enthält.

5. Verfahren nach einem oder mehreren der Ansprüche 1 bis 4, dadurch gekennzeichnet, daß

a) die Zugabe der sauren Lösung (A) von Butylglucosid in Butanol zum erhitzten Alkohol oder Alkohol/Katalysator-Gemisch (B) kontinuierlich erfolgt,

b) gleichzeitig Butanol im Vakuum aus dem Reaktionsgemisch destillativ entfernt wird und

c) bei Verwendung eines Alkohol-Katalysator-Gemisches (B) in beiden sauren Reaktionskomponenten derselbe Katalysator, vorzugsweise Paratoluolsulfonsäure, eingesetzt wird.

6. Erzeugnis erhältlich nach dem Verfahren nach einem oder mehreren der Ansprüche 1 bis 5, dadurch gekennzeichnet, daß der mittlere Oligomerisierungsgrad der Alkylglucoside 1,1 bis 1,4, vorzugsweise etwa 1,3 beträgt.

7. Erzeugnis nach Anspruch 6, dadurch gekennzeichnet, daß es im wesentlichen aus

0,5 bis 5, vorzugsweise 2,5 bis 4 Gew.-% Fettalkohol,

55 bis 90, vorzugsweise 60 bis 90 Gew.-% Alkylmonoglucosid,

2 bis 22, vorzugsweise 3 bis 20 Gew.-% Alkyloligoglucosid,

3 bis 10, vorzugsweise 5 bis 7 Gew.-% Butylglucosid und

4 bis 25 Gew.-% Polyglucose besteht.

| Europäisches Patentamt | EUROPÄISCHER RECHERCHENBERICHT | Nummer der Anmeldung |
| --- | --- | --- |
| | | EP 89 12 2750 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| --- | --- | --- | --- |
| X,D | EP-A-0 092 875 (PROCTER & GAMBLE) <br> * Insgesamt * <br> --- | 1-7 | C 07 H 15/04 |
| X | DE-A-1 905 523 (ATLAS) <br> * Insgesamt * <br> --- | 1-7 | |
| X | FR-A-1 411 864 (RÖHM & HAAS) <br> * Insgesamt * <br> --- | 1-7 | |
| A | FR-A-2 017 240 (RÖHM & HAAS) <br> * Patentansprüche 1-8 * & US-A-3 547 828 (Kat. D) <br> ----- | 6,7 | |
| | | | **RECHERCHIERTE SACHGEBIETE (Int. Cl.5)** |
| | | | C 07 H 15/00 |

Der vorliegende Recherchenbericht wurde für alle Patentansprüche erstellt

| Recherchenort | Abschlußdatum der Recherche | Prüfer |
| --- | --- | --- |
| DEN HAAG | 23-03-1990 | BRENNAN J. |